# EUROPÄISCHE PATENTSCHRIFT

(11) **EP 1 948 125 B1**
(45) Veröffentlichungstag und Bekanntmachung des Hinweises auf die Patenterteilung: **18.06.2014**
(21) Anmeldenummer: 06792398.7
(22) Anmeldetag: 07.10.2006
(51) Int. Cl.: A61K 8/42, A61Q 5/06, A61Q 5/08, A61Q 5/10, A61Q 5/12

(54) **VOLUMENGEBENDES HAARBEHANDLUNGSMITTEL MIT HYDROXYALKYLHARNSTOFF**
VOLUME ENHANCING HAIR CARE PREPARATION
AGENT DE TRAITEMENT CAPILLAIRE VOLUMISANT

(30) Priorität: 16.11.2005 DE 102005054974
(43) Veröffentlichungstag der Anmeldung: 30.07.2008
(73) Patentinhaber: Henkel AG & Co. KGaA, 40589 Düsseldorf (DE)
(72) Erfinder: SEILER, Martina, 47228 Duisburg (DE); MIEHLICH, Kristin, 42119 Wuppertal (DE)
(86) Internationale Anmeldenummer: PCT/EP2006/009709
(87) Internationale Veröffentlichungsnummer: WO 2007/057073

(56) Entgegenhaltungen:
- EP-A- 1 535 607
- EP-A- 1 600 147
- EP-A- 1 671 680
- WO-A-2005/079740
- WO-A-2006/018198
- WO-A-2006/042626
- WO-A-2006/056692
- DE-A1- 2 703 185
- DE-A1- 10 154 772
- Wilfried Umbach: "Kosmetik und Hygiene", Wiley-VCH Verlag GmbH & Co. KGaA, Weinheim, Düsseldorf ISBN: 3-527-30996-9 page 106, 109, 294, * the whole document *

## Beschreibung

Die vorliegende Erfindung betrifft die Verwendung von N-Hydroxyalkyl-substituierten Harnstoffderivaten zur Verdickung keratinhaltiger Fasern, insbesondere menschlicher Haare, sowie ein Verfahren zur Verdickung keratinhaltiger Fasern, insbesondere menschlicher Haare.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten.

Das Haar bzw. die Frisur kann im Laufe der Zeit, insbesondere durch oxidativ und reduktiv wirkende kosmetische Behandlungen, an Struktur und Fülle bzw. Volumen verlieren. Manche Menschen besitzen von Natur aus dünnes Haar, wodurch der Wunsch nach einer füllig erscheinenden Frisur genährt wird.

Voluminöses und gesund erscheinendes Haar bleibt nachweislich ein Schönheitsideal. Kosmetika, die keratinhaltigen Fasern möglichst dauerhaft einen Volumenzuwachs bescheren sind rar. Folglich besteht ein dringendes Bedürfnis nach entsprechenden Kosmetika.

Aufgabe der vorliegenden Erfindung ist es daher, das Volumen keratinhaltiger Fasern möglichst dauerhaft zu erhöhen.

Es wurde nun überraschenderweise gefunden, daß sich das Volumen keratinhaltiger Fasern durch die Verwendung N-Hydroxyalkyl-substituierter Harnstoff-Derivate erhöhen lässt. Dies kann sogar während der oxidativen Haarbehandlung geschehen. Unter keratinhaltigen Fasern werden erfindungsgemäß Pelze, Wolle tierischen Ursprungs, Federn und insbesondere menschliche Haare verstanden.

Ein erster Gegenstand der vorliegenden Erfindung ist daher die Verwendung von Harnstoffderivaten gemäß Formel I, 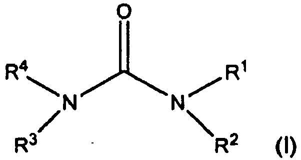 worin
die Reste R¹, R², R³ und R⁴ unabhängig voneinander für ein Wasserstoffatom, eine C₁- bis C₄-Alkylgruppe, eine C₂- bis C₆-Alkenylgruppe oder eine C₂- bis C₆-Hydroxyalkylgruppe mit mindestens einer Hydroxygruppe steht,
mit der Maßgabe, dass mindestens einer der besagten Reste eine C₂- bis C₆-Hydroxyalkylgruppe mit mindestens einer Hydroxygruppe bedeutet,
zur Erhöhung des Volumens keratinhaltiger Fasern, insbesondere menschlicher Haare, dadurch gekennzeichnet, dass das kosmetische Mittel, das das Harnstoffderivat gemäß Formel (I) enthält zusätzlich als farbverandernde komponente mindestens ein Oxidationsfarbstoffvorprodukt vom Typ der Entwicklerkomponente und gegebenenfalls zusätzlich mindestens eine Kupplerkomponente und mindestens ein Oxidationsmittel enthält.

Als bevorzugt geeignete Harnstoffderivate der Formel (I) dienen solche, die mindestens eine C₂- bis C₆-Hydroxyalkylgruppe mit mindestens einer Hydroxygruppe enthalten, welche aus mindestens einem Vertreter aus der Gruppe aus 2-Hydroxyethyl, 2-Hydroxy-2-methylprop-2-yl, 1,3-Dihydroxy-2-methylprop-2-yl, 1,3-Dihydroxyprop-2-yl, Tris(hydroxymethyl)methyl, 1,3-Dihydroxy-2-hydroxymethylprop-2-yl, 2,3-Dihydroxypropyl, 2-Hydroxypropyl, 3-Hydroxypropyl, 4-Hydroxybutyl, 1-Hydroxy-2-methylprop-2-yl, 2,3,4,5,6-Pentahydroxyhexyl und 1,3,4,5,6-Pentahydroxyhex-2-yl ausgewählt wird.

Es ist erfindungsgemäß bevorzugt solche Harnstoffderivate der Formel (I) zu verwenden, welche die bevorzugte Maßgabe der Formel (I) erfüllen, daß genau einer der Reste R¹ bis R⁴ eine C₂- bis C₆-Hydroxyalkylgruppe mit mindestens einer Hydroxygruppe bedeutet.

Beispiele für erfindungsgemäß in Verbindungen der Formel (I) verwendbare C₁- bis C₄-Alkylgruppen sind Methyl, Ethyl, n-Propyl, Isopropyl, n-Butyl, sec-Butyl, Isobutyl und tert-Butyl.

Beispiele für erfindungsgemäß in Verbindungen der Formel (I) verwendbare C₂- bis C₆-Alkenylgruppen sind Vinyl, 2-Propen-1-yl (Allyl) oder 3-Buten-1-yl.

Besonders bevorzugt wird mindestens eine Verbindung aus der Gruppe mit den Vertretern, N-(2-Hydroxyethyl)harnstoff, N-(3-Hydroxypropyl)harnstoff, N-(2-Hydroxypropyl)harnstoff, N-(2-Hydroxy-2-methyl-prop-2 -yl)harnstoff, N-(1,3-Dihydroxy-2-methyl-prop-2-yl)harnstoff und N-(1,3-Dihydroxy-2-hydroxymethylprop-2-yl)harnstoff ausgewählt. Ganz besonders bevorzugt ist N-(2-Hydroxyethyl)harnstoff, erhältlich beispielsweise als Handelsprodukt Hydrovance^{®} von der Firma National Starch.

Die Verbindungen gemäß Formel (I) werden bevorzugt durch Applikation eines die besagten Verbindungen enthaltenen kosmetischen Mittels auf die keratinhaltige Faser aufgebracht, wobei das besagte kosmetische Mittel zusätzlich als farbverändernde komponente mindestens ein Oxidationsfarbstoffvorprodukt vom Typ der Entwicklerkomponenten und gegebenenfalls zusätzlich mindestens eine Kupplerkomponente und mindestens ein Oxidationsmittel enthält.

Ein zweiter Gegenstand der Erfindung ist ein Verfahren zur Volumensteigerung keratinhaltiger Fasern, insbesondere menschlicher Haare, in dem ein kosmetisches Mittel, enthaltend mindestens ein Harnstoffderivat gemäß Formel (I) 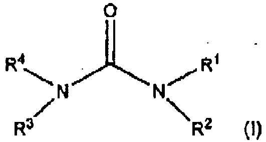 worin
die Reste R¹, R², R³ und R⁴ wie im ersten Erfindungsgegenstand definiert sind, auf die keratinhaltigen Fasern aufgetragen und gegebenenfalls nach einer Einwirkungszeit wieder abgespült wird, wobei das besagte kosmetische Mittel zusätzlich als farbverändernde komponente mindestens ein Oxidationsfarbstoffvorprodukt vom Typ der Entwicklerkomponenten und gegebenenfalls zusätzlich mindestens eine Kupplerkomponente und mindestens ein Oxidationsmittel enthält.

Das kosmetische Mittel des erfindungsgemäßen Verfahrens enthält die Harnstoffderivate der Formel (I) in einem kosmetischen Träger. Als kosmetische Träger eignen sich besonders Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind. Es ist aber auch denkbar, die Inhaltsstoffe in eine pulverförmige oder auch tablettenförmige Formulierung zu integrieren, welche vor der Anwendung in Wasser gelöst wird. Die kosmetischen Träger können insbesondere wässrig oder wässrig-alkoholisch sein.

Ein wässriger kosmetischer Träger enthält mindestens 50 Gew.-% Wasser.

Unter wässrig-alkoholischen kosmetischen Trägern sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die kosmetischen Mittel des Verfahrens enthalten die Harnstoffderivate gemäß Formel (I) bevorzugt in einer Menge von 0,05 bis 5 Gew.-%, besonders bevorzugt von 0,25 bis 2,5 Gew.-%, jeweils bezogen auf das Gewicht des anwendungsbereiten Mittels.

Besonders bevorzugt wird das kosmetische Mittel des erfindungsgemäßen Verfahrens auf das Kopfhaar aufgetragen.

Die Einwirkungszeit beträgt bevorzugt 1 bis 100 Minuten, besonders bevorzugt 5 bis 50 Minuten.

Das kosmetische Mittel kann als rinse-off oder leave-on Produkt formuliert werden. Es ist erfindungsgemäß bevorzugt, das kosmetische Mittel nach Ablauf der Einwirkungszeit von der keratinhaltigen Faser zu spülen.

Das erfindungsgemäß Verfahren kann vor oder nach einer herkömmlichen Haarbehandlung, wie Färbung, Tönung, Bleiche, temporärem Styling oder Dauerwelle, durchgeführt werden. Das erfindungsgemäße Verfahren kann sogar in einen Verfahrensschritt eines herkömmlichen Haarbehandlungsverfahrens integriert werden. All dies gilt insbesondere für Verfahren zu Farbveränderung und für oxidative Haarbehandlungsverfahren.

Wie erwähnt ist es bevorzugt, das erfindungsgemäße Verfahren im Rahmen einer Farbveränderung der keratinhaltigen Faser, insbesondere in Kombination mit einer oxidativen Faserbehandlung, durchzuführen. Zu diesem Zweck enthalten die kosmetischen Mittel des erfindungsgemäßen Verfahrens zusätzlich mindestens eine farbverändernde Komponente.

Die farbverändernde Komponente wird wiederum bevorzugt ausgewählt
(a) aus mindestens einem Oxidationsfarbstoffvorprodukt vom Typ der Entwicklerkomponenten und gegebenenfalls zusätzlich mindestens einer Kupplerkomponente
   und/oder
(b) aus Oxofarbstoffvorprodukten
   und/oder
(c) aus mindestens einem direktziehenden Farbstoff
   und/oder
(d) aus mindestens einer Vorstufe naturanaloger Farbstoffe
   und/oder
(e) aus mindestens einem Oxidationsmittel und gegebenenfalls zusätzlich mindestens einem Bleichverstärker.

Als Entwicklerkomponenten werden üblicherweise primäre aromatische Amine mit einer weiteren, in para- oder ortho-Position befindlichen, freien oder substituierten Hydroxyoder Aminogruppe, Diaminopyridinderivate, heterozyklische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (Ent1) 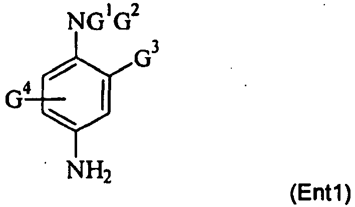 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁-bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (Ent1) sind insbesondere die Aminogruppen, C₁-bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (Ent1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin-, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (Ent1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Mitteln gemäß der Erfindung verwendet werden können, kann man insbesondere die Verbindungen nennen, die der folgenden Formel (Ent2) entsprechen, sowie ihre physiologisch verträglichen Salze: 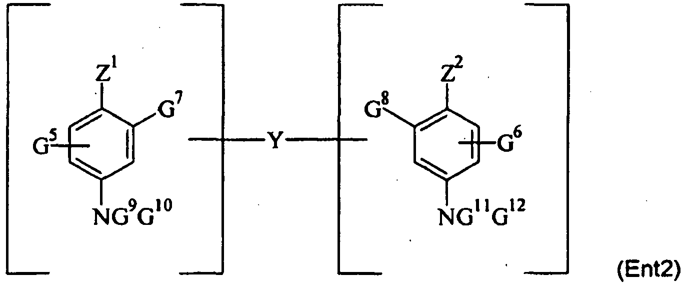 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
   mit den Maßgaben, dass
- die Verbindungen der Formel (Ent2) nur eine Verbrückung Y pro Molekül enthalten und
- die Verbindungen der Formel (Ent2) mindestens eine Aminogruppe enthalten, die mindestens ein Wasserstoffatom trägt.

Die in Formel (Ent2) verwendeten Substituenten sind erfindungsgemäß analog zu den obigen Ausführungen definiert.

Bevorzugte zweikernige Entwicklerkomponenten der Formel (Ent2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (Ent2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (Ent3) 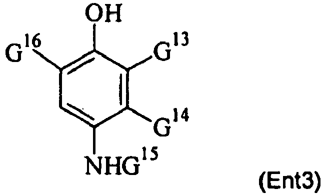 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoky-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁-bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁-bis C₄)-Alkoxy-(C₁- bis C₄)-alkylrest, einen C₁- bis C₄-Aminoalkylrest oder einen C₁-bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (Ent3) verwendeten Substituenten sind erfindungsgemäß analog zu den obigen Ausführungen definiert.

Bevorzugte p-Aminophenole der Formel (Ent3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(ß-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (Ent3) sind p-Amiriophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamlno-pyridin, 2-(4'-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(ß-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)amino-1-methylpyrazol.

Bevorzugte Pyrazolopyrimidin-Derivate sind insbesondere die Derivate des Pyrazolo[1,5-a]opyrimidin der folgenden Formel (Ent4) und dessen tautomeren Formen, sofern ein tautomeres Gleichgewicht besteht: 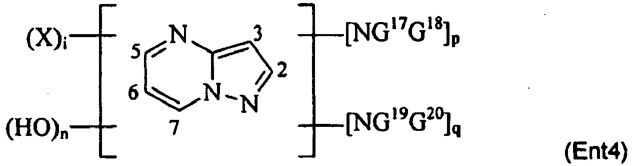 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁-bis C₄-hydroxyalkyl)aminoalkylrest, einen Aminorest, einen C₁- bis C₄-Alkyl- oder Di-(C₁- bis C₄-hydroxyalkyl)aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
   mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (Ent4) verwendeten Substituenten sind erfindungsgemäß analog zu den obigen Ausführungen definiert.

Wenn das Pyrazolo[1,5-a]pyrimidin der obenstehenden Formel (Ent4) eine Hydroxygruppe an einer der Positionen 2, 5 oder 7 des Ringsystems enthält, besteht ein tautomeres Gleichgewicht, das zum Beispiel im folgenden Schema dargestellt wird:

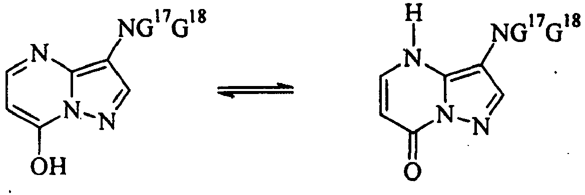

Unter den Pyrazolo[1,5-a]pyrimidinen der obenstehenden Formel (Ent4) kann man insbesondere nennen:
- Pyrazolo[1,5-a]pyrimidin-3,7-diamin;
- 2,5-Dimethyl-pyrazolo[1,5-a]pyrimidin-3,7-diamin;
- Pyrazolo[1,5-a]pyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-yl)-(2-hydroxy-ethyl)amino]-ethanol;
- 2-[(7-Aminopyrazolo[1,5-a]pyrimidin-3-yl)-(2-hydroxy-ethyl)amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;
sowie ihre physiologisch verträglichen Salze und ihre tautomeren Formen, wenn ein tautomeres Gleichgewicht vorhanden ist.

Die Pyrazolo[1,5-a]pyrimidine der obenstehenden Formel (Ent4) können wie in der Literatur beschrieben durch Zyklisierung ausgehend von einem Aminopyrazol oder von Hydrazin hergestellt werden.

Als Kupplerkomponenten werden in der Regel m-Phenylendiaminderivate, Naphthole, Resorcin und Resorcinderivate, Pyrazolone und m-Aminophenolderivate sowie heterozyklische Verbindungen verwendet.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}-amino)ethanol, 2-[3-Morpholin-4-ylphenyl)amino]ethanol, 3-Amino-4-(2-methoxy-ethoxy)-5-methylphenylamin und 1-Amino-3-bis-(2'-hydroxyethyl)-aminobenzol,

- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin und die physiologisch verträglichen Salzen der vorgenannten Verbindungen.

Die kosmetischen Mittel des erfindungsgemäßen Verfahrens enthalten die Entwicklerkomponenten bevorzugt in einer Menge von 0,005 bis 10 Gew.-%, vorzugsweise von 0,1 bis 5 Gew.-%, jeweils bezogen auf das gesamte Mittel.

Die kosmetischen Mittel des erfindungsgemäßen Verfahrens enthalten die Kupplerkomponenten bevorzugt in einer Menge von 0,005 bis 10 Gew.-%, vorzugsweise von 0,1 bis 5 Gew.-%, jeweils bezogen auf das gesamte Mittel.

Das Mittel des erfindungsgemäßen Verfahrens kann als farbverändernde Komponente in Form der Oxofarbstoffvorprodukte mindestens eine Kombination, aus mindestens einer Verbindung der Komponente
1 Verbindungen, die eine reaktive Carbonylgruppe enthalten mit mindestens einer Verbindung der Komponente
2 Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen
enthalten.

Erfindungsgemäße Verbindungen mit einer reaktiven Carbonylgruppe (im Folgenden auch reaktive Carbonylverbindungen oder Komponente 1 genannt) besitzen mindestens eine Carbonylgruppe als reaktive Gruppe, welche mit den Verbindungen der Komponente 2 unter Ausbildung einer beide Komponenten verknüpfenden chemischen Bindung reagiert. Ferner sind erfindungsgemäß auch solche Verbindungen als Komponente 1 umfaßt, in denen die reaktive Carbonylgruppe derart derivatisiert bzw. maskiert ist, daß die Reaktivität des Kohlenstoffatoms der derivatisierten bzw. maskierten Carbonylgruppe gegenüber der Komponente 2 stets vorhanden ist. Diese Derivate sind bevorzugt Kondensationsverbindungen von reaktiven Carbonylverbindungen mit
a) Aminen und deren Derivate unter Bildung von Iminen oder Oximen als Kondensationsverbindung
b) Alkoholen unter Bildung von Acetalen oder Ketalen als Kondensationsverbindung
c) Wasser unter Bildung von Hydraten als Kondensationsverbindung von Aldehyden.

Die Komponente 1 wird bevorzugt ausgewählt aus der Gruppe, die gebildet wird aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxyacetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbon-säure, Acetophenon-4-carbonsäure, Benzophenon, 4-Hydroxybenzophenon, 2-Aminobenzophenon, 4,4'-Dihydroxybenzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron, Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxy-benzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxybenzaldehyd, 4-Hydroxy-3,5-dimethyl-benzaldehyd, 3,5-Diethoxy-4-hydroxybenzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxybenzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaidehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,5-Dihydroxybenzaldehyd, 2,6-Dihydroxybenzaldehyd, 3,4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 2-Methoxy-zimtaldehyd, 4-Methoxy-zimtaldehyd, 4-Hydroxy-3-methoxy-zimtaldehyd, 3,5-Dimethoxy-4-hydroxyzimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylaminozimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd, Piperonal, 2,3,6,7-Tetrahydro-1H,5H-benzo[ij]chinolizin-9-carboxaldehyd, 2,3,6,7-Tetrahydro-8-hydroxy-1 H,5H-benzo[ij]chinolizin-9-carboxaldehyd, N-Ethylcarbazol-3-aldehyd, 2-Formyl-methylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indoli-nyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon, Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)hexa-3,5-dien-2-on, 6-(4-Pyrrolidinophenyl)hexa-3,5-dien-2-on, 5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-meth-oxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazolium-Salze, bevorzugt mit Benzolsulfonat, p-Toluolsulfonat, Methansulfonat, Perchlorat, Sulfat, Chlorid, Bromid, Iodid, Tetrachlorozinkat, Methylsulfat, Trifluormethansulfonat oder Tetrafluoroborat als Gegenion, Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Als CH-acide werden im allgemeinen solche Verbindungen angesehen, die ein an ein aliphatisches Kohlenstoffatom gebundenes Wasserstoffatom tragen, wobei aufgrund von elektronenziehenden Substituenten eine Aktivierung der entsprechenden Kohlenstoff-Wasserstoff-Bindung bewirkt wird. Unter CH-acide Verbindungen fallen erfindungsgemäß auch Enamine, die durch alkalische Behandlung von quaternierten N-Heterozyklen mit einer in Konjugation zum quartären Stickstoff stehenden CH-aciden Alkylgruppe entstehen.

Die CH-aciden Verbindungen der Komponente 2 sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitur-säure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

Bevorzugte primäre oder sekundäre aromatische Amine der Komponente 2 sind ausgewählt aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-l-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxybenzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)aminol-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-pheny-lendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest, wie sie in der Formel II dargestellt sind 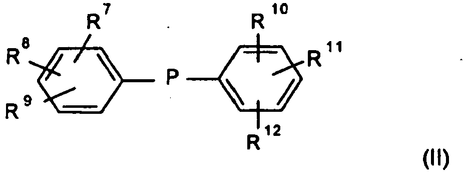 in der
- R⁷ für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht,
- R⁸, R⁹, R¹⁰, R¹¹ und R¹² unabhängig voneinander für ein Wasserstoffatom, eine Hydroxy- oder eine Aminogruppe, die durch C₁-C₄-Alkyl-, C₁-C₄-Hydroxyalkyl, C₁-C₄-Alkoxy-, C₁-C₄-Aminoalkyl- oder C₁-C₄-Alkoxy-C₁-C₄-alkylgruppen substituiert sein kann, stehen, und
- P für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfoxy-, Sulfonyl- oder Iminogruppe, ein Sauerstoff- oder Schwefelatom, oder eine Gruppe mit der Formel III

   -Q'-(CH₂-Q-CH₂-Q")ₒ- (III)

   in der
   - Q eine direkte Bindung, eine CH₂- oder CHOH-Gruppe bedeutet,
   - Q' und Q" unabhängig voneinander für ein Sauerstoffatom, eine NR¹³-Gruppe, worin R¹³ ein Wasserstoffatom, eine C₁₋₄-Alkyl- oder eine Hydroxy-C₁₋₄-alkylgruppe, wobei auch beide Gruppen zusammen mit dem Restmolekül einen 5-, 6- oder 7-Ring bilden können, bedeutet, die Gruppe O-(CH₂)ₚ-NH oder NH-(CH₂)ₚ-O, worin p und p' 2 oder 3 sind, stehen und
   - o eine Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methylpyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Amino-isochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin und 4-Hydroxyindolin. Weiterhin als heterocyclische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylaminophenol, 2-(2-Hydroxyethyl)phenol, 3,4-Methylendioxyphenol, 2,4-Dihydroxybenzoesäure, 3,4-Dihydroxybenzoesäure, 2,4-Dihydroxy-phenylessigsäure, 3,4-Dihydroxy-phenylessigsäure, Gallussäure, 2,4,6-Trihydroxybenzoesäure, 2,4,6-Trihydroxyacetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure.

Die Verbindungen der Komponente 1 und die Verbindungen der Komponente 2 werden vorzugsweise in den kosmetischen Mitteln jeweils in einer Menge von 0,03 bis 65 mmol, insbesondere von 1 bis 40 mmol, bezogen auf 100 g des gesamten Nuanciermittels, verwendet. Das molare Verhältnis von der Verbindung der Komponente 1 und der Verbindung der Komponente 2 kann im Bereich von 0,5 bis 2,0 liegen, wobei vorzugsweise äquimolare Mengen eingesetzt werden. Das anwendungsbereite Mittel wird bei getrennter Lagerung der Komponenten 1 und 2 unmittelbar vor der Anwendung durch Mischen hergestellt.

Als Vorstufen naturanaloger Farbstoffe werden bevorzugt als Oxidationsfarbstoffvorprodukt vom Entwicklertyp solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (IVa), 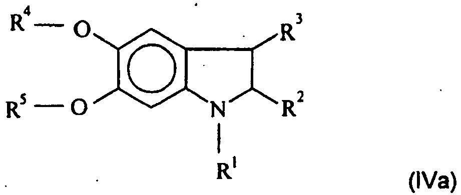 in der unabhängig voneinander
- R¹ steht für Wasserstoff, eine C₁-C₄-Alkylgruppe oder eine C₁-C₄-Hydroxy-alkyl-gruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IVb), 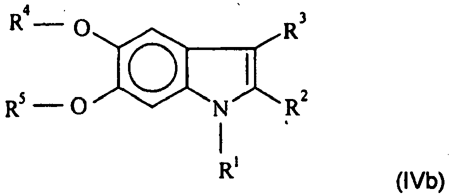 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Bevorzugte direktziehende Farbstoffe, die in den kosmetischen Mitteln des erfindungsgemäßen Verfahrens als farbverändernde Komponente Verwendung finden, sind Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die kosmetischen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

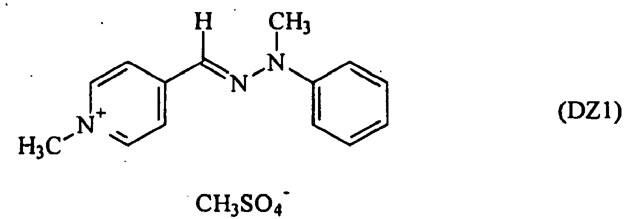

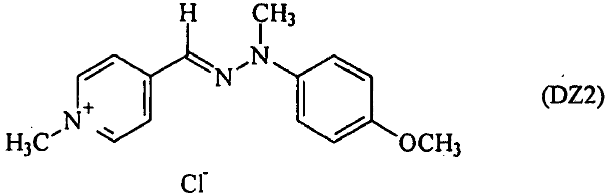

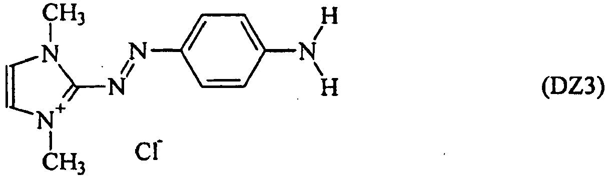

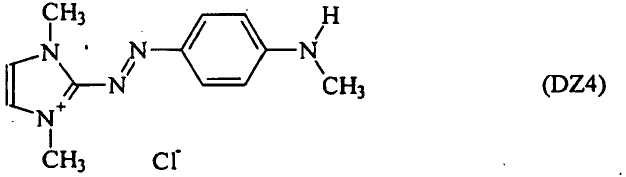

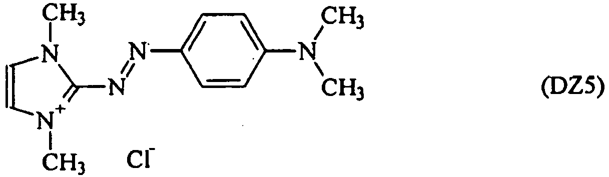

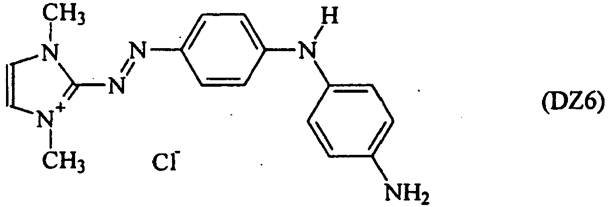

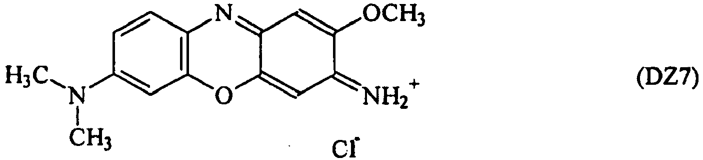

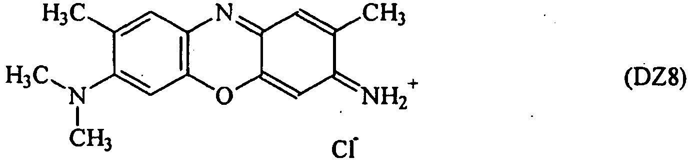

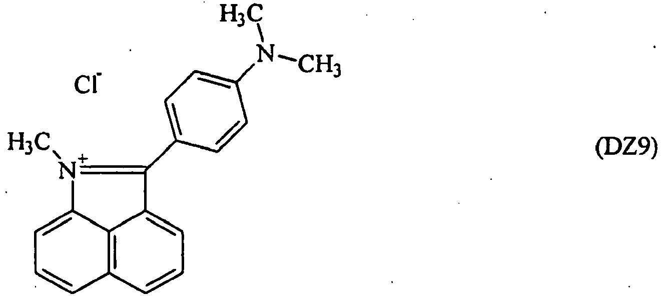

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die kosmetischen Mittel des erfindungsgemäßen Verfahrens enthalten die direktziehenden Farbstoffe bevorzugt in einer Menge von 0,01 bis 20 Gew.-%, bezogen auf das anwendungsbereite Mittel.

Weiterhin können die kosmetischen Mittel des erfindungsgemäßen Verfahrens auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den kosmetischen Mitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Eine oxidative Färbung kann in Gegenwart von Oxidationsfarbstoffvorprodukten grundsätzlich mit Luftsauerstoff erfolgen. Bevorzugt wird jedoch zusätzlich ein von Luftsauerstoff verschiedenes Oxidationsmittel eingesetzt, besonders dann, wenn neben der Färbung ein Aufhelleffekt der natürlichen Pigmente des menschlichen Haars gewünscht ist. Dieser Aufhelleffekt kann unabhängig von der Färbemethode gewünscht sein. Die Gegenwart von Oxidationsfarbstoffvorprodukten ist demnach keine zwingende Voraussetzung für einen Einsatz von Oxidationsmitteln in den kosmetischen Zusammensetzungen des erfindungsgemäßen Verfahrens.

Als Oxidationsmittel kommt insbesondere Wasserstoffperoxid und/oder mindestens ein Anlagerungsprodukt davon, insbesondere an anorganische oder organische Verbindungen, wie beispielsweise Natriumperborat, Natriumpercarbonat, Magnesiumpercarbonat, Natriumpercarbamid, Polyvinylpyrrolidon·n H₂O₂ (n ist eine positive ganze Zahl größer 0), Harnstoffperoxid und Melaminperoxid in Frage. Das eigentliche Färbemittel wird bei getrennter Lagerung der Farbstoffvorprodukte und des Oxidationsmittels unmittelbar vor der Anwendung durch Mischen hergestellt. In einer bevorzugten Ausführungsform des erfindungsgemäßen Verfahrens wird daher das kosmetische Mittel vor der Applikation aus einer Zusammensetzung, enthaltend in einem kosmetischen Träger mindestens eine farbverändernde Komponente, und einer weiteren Zusammensetzung, enthaltend in einem kosmetischen Träger mindestens ein Oxidationsmittel, gemischt.

Erfindungsgemäß kann das kosmetische Mittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme. Die Katalysatoren können auch in Gegenwart eines Oxidationsmittels verwendet werden.

Geeignete·Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Was-serstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Bei einer Anwendung von Oxidationsmitteln wird die anwendungsfertige Zubereitung zweckmäßigerweise unmittelbar vor der Anwendung durch Mischung einer Zusammensetzung, enthaltend das Oxidationsmittel mit der Zusammensetzung, enthaltend die farbverändernden Komponenten, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Färbemittel in einem schwach alkalischen Milieu.

Für eine Farbveränderung mittels Aufhellung bzw. Bleichung der Haare wird bevorzugt neben den Oxidationsmitteln zusätzlich mindestens ein Bleichverstärker eingesetzt.

Bleichverstärker werden bevorzugt in Blondiermitteln zur Steigerung der Blondierwirkung des Oxidationsmittels, insbesondere des Wasserstoffperoxids, eingesetzt.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Als Bleichverstärker können erfindungsgemäß bevorzugt Carbonatsalze, bzw. Hydrogencarbonatsalze eingesetzt werden. Diese sind bevorzugt ausgewählt aus der Gruppe der Ammonium-, Alkali- (insbesondere Natrium- und Kalium-), sowie Erdalkali-(insbesondere Calcium-), -carbonatsalze bzw. -hydrogencarbonatsalze. Besonders bevorzugte Carbonat-, bzw. Hydrogencarbonatsalze sind Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat. Diese besonders bevorzugten Salze können allein oder in deren Mischungen von mindestens zwei Vertretern als Bleichverstärker verwendet werden.

Als Bleichverstärker vom Typ der Monoalkylcarbonate und deren Derivate werden bevorzugt mindestens ein Kohlensäuremonoester und/oder mindestens ein Kohlensäuremonoamid in dem erfindungsgemäßen Verfahren verwendet. Bevorzugt verwendbare Kohlensäuremonoester sind die Kohlensäuremonoester der Formel (V), 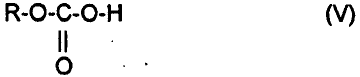 in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (V) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugt verwendete Zusammensetzungen sind dadurch gekennzeichnet, daß der Rest R in Formel (V) ausgewählt ist aus Methyl-, Ethyl-, n-Propyl-, iso-Propyl-, n-Butyl-, iso-Butyl-, *tert*-Butyl- sowie Hydroxymethyl- und Hydroxyethyl-Resten.

Alternativ zum Kohlensäuremonoester oder in Verbindung mit ihm können in den wasserfreien Zusammensetzungen Kohlensäuremonoamide als Bleichverstärker eingesetzt werden. Hier ist es erfindungsgemäß bevorzugt, mindestens ein Kohlensäuremonoamid der Formel (VI) zu verwenden, 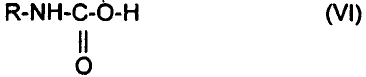 in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (VI) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere Bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugte Bleichverstärker der Formel (VI) sind dadurch gekennzeichnet, daß der Rest R in Formel (VI) ausgewählt ist aus Methyl-, Ethyl-, n-Propyl-, iso-Propyl-, n-Butyl-, iso-Butyl-, *tert*-Butyl- sowie Hydroxymethyl- und Hydroxyethyl-Resten.

Das acide H-Atom des Kohlensäuremonoesters bzw. -monoamids kann auch in neutralisierter Form vorliegen, d.h. es können erfindungsgemäß auch Salze von Kohlensäuremonoestern bzw. Kohlensäuremonoamiden eingesetzt werden. Hier sind erfindungsgemäß Kohlensäuremonoester bzw. das Kohlensäuremonoamide bevorzugt, die in ganz oder teilweise neutralisierter Form, vorzugsweise in Form des Alkalimetall-, Ammonium-, Erdalkalimetall- oder Aluminiumsalzes und insbesondere in Form seines Natriumsalzes, vorliegen.

Als Bleichverstärker vom Typ der Silylcarbonate und deren Derivate werden bevorzugt mindestens ein Silylcarbonat und/oder mindestens ein Silylcarbamat in die erfindungsgemäßen Zusammensetzungen eingearbeitet. Bevorzugt werden Silylcarbonate gemäß Formel (VII) eingesetzt, 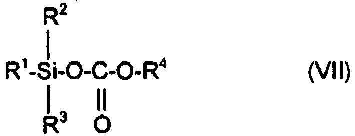 in der die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstituierte Arylgruppe bzw. einen substituierten oder unsubstituierten Heterocyclus oder für ein Halogen, eine substituierte oder unsubstituierte Hydroxy-, Oxo-Aminogruppen stehen und der Rest R⁴ für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstituierte Arylgruppe bzw. einen substituierten oder unsubstituierten Heterocyclus steht.

Bevorzugte Reste R¹, R² und R³ in der oben genannten Formel (VII) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugte erfindungsgemäße wasserfreie Zusammensetzungen dadurch gekennzeichnet sind, daß die Reste R¹, R² und R³ in Formel (VII) ausgewählt sind aus Methyl-, Ethyl-, n-Propyl-, iso-Propyl-, n-Butyl-, iso-Butyl-, *tert*-Butyl- sowie Hydroxymethyl- und Hydroxyethyl-Resten.

Bevorzugte Reste R⁴ in der oben genannten Formel (VII) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Als Bleichverstärker kann mindestens ein Silylcarbamat der Formel (VIII) in der erfindungsgemäßen wasserfreien Zusammensetzung enthalten sein, 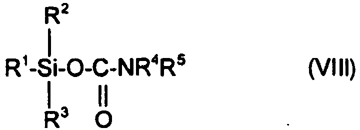 wobei die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstituierte Arylgruppe bzw. einen substituierten oder unsubstituierten Heterocyclus oder für ein Halogen, eine substituierte oder unsubstituierte Hydroxy-, Oxo-Aminogruppen stehen und die Rest R⁴ und R⁵ unabhängig voneinander für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstituierte Arylgruppe bzw. einen substituierten oder unsubstituierten Heterocyclus stehen.

Bevorzugte Reste R¹, R² und R³ in der oben genannten Formel (VIII) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugt verwendete Zusammensetzungen dadurch gekennzeichnet sind, daß die Reste R¹, R² und R³ in Formel (VIII) ausgewählt sind aus Methyl-, Ethyl-, n-Propyl-, iso-Propyl-, n-Butyl-, iso-Butyl-, *tert*-Butyl- sowie Hydroxymethyl- und Hydroxyethyl-Resten.

Bevorzugte Reste R⁴ und R⁵ in der oben genannten Formel (VIII) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Als weitere zusätzliche Bleichverstärker können in den erfindungsgemäßen Zusammensetzungen bevorzugt mindestens eine Verbindung ausgewählt aus Essigsäure, Milchsäure, Weinsäure, Zitronensäure, Salicylsäure und ortho-Phthalsäure, enthalten sein.

Bleichverstärker sind bevorzugt Peroxoverbindungen. Unter die erfindungsgemäß bleichverstärkenden Peroxoverbindungen fallen keine Anlagerungsprodukte von Wasserstoffperoxid an andere Komponenten und auch nicht Wasserstoffperoxid selbst. Die Auswahl der Peroxoverbindungen unterliegt darüber hinaus keinen Beschränkungen. Bevorzugte Peroxoverbindungen sind Peroxidisulfatsalze, Persulfatsalze, (insbesondere Ammoniumperoxidisulfat, Kaliumperoxidisulfat, Natriumperoxidisulfat, Ammoniumpersulfat, Kaliumpersulfat, Natriumpersulfat, Kaliumperoxidiphosphat) und Peroxide (wie Bariumperoxid und Magnesiumperoxid). Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Ammoniumperoxidisulfat.

Die Bleichverstärker sind in den kosmetischen Mitteln des erfindungsgemäßen Verfahrens bevorzugt in Mengen von 5-30 Gew.-%, insbesondere in Mengen von 8-20 Gew.-%, enthalten.

Die kosmetischen Zusammensetzungen des erfindungsgemäßen Verfahrens enthalten, wenn sie als Bleichmittel fungieren, als bevorzugtes Alkalisierungsmittel mindestens eine Verbindung, ausgewählt aus Ammonium-, Alkalimetall- und Erdalkalimetallhydroxiden, - carbonaten, -hydrogencarbonaten, -hydroxycarbonaten, -metasilikaten und -carbamiden, sowie Alkaliphosphaten.

Die in dem erfindungsgemäßen Verfahren eingesetzten kosmetischen Mittel können weiterhin alle für solche Zubereitungen bekannten Wirk-, Zusatz- und Hilfsstoffe enthalten:

Das Nachwaschen mit einem Shampoo entfällt, wenn ein stark tensidhaltiger Träger verwendet wurde.

In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in den kosmetischen Mitteln alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder PolygJykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyldimethylamin dar.

Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die kosmetischen Mittel des erfindungsgemäßen Verfahrens weitere Wirk-, Hilfs- und Zusatzstoffe enthalten, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-tri-methylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessemde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein dritter Erfindungsgegenstand ist ein Mittel, enthaltend in einem kosmetischen Träger eine Kombination aus mindestens einem Harnstoffderivat gemäß Formel (I) 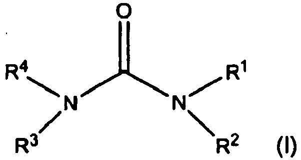 wobei die Reste R¹, R², R³ und R⁴ wie im ersten Erfindungsgegenstand definiert sind und mindestens einer farbverändernden Komponente, wobei die farbverändernde Komponente mindestens ein Oxidationsfarbstoffvorprodukt vom Typ des Entwicklerkomponenten und gegebenenfalls zusätzlich mindestens eine Kupplerkomponente und mindestens ein Oxidationsmittel enthält.

Alle bevorzugten Ausführungsformen und optionalen Bestandteile des kosmetischen Mittels des Verfahrens gemäß zweitem Erfindungsgegenstand gelten ebenso für das erfindungsgemäße Mittel des dritten Erfindungsgegenstandes.

Die folgenden Beispiele sollen den Gegenstand der vorliegenden Erfindung erläutern ohne ihn jedoch zu beschränken.

### Beispiele

### 1.0 Herstellung von Cremegrundlagen

Alle Mengenangaben erfolgen, falls nicht anders angegeben, in Gew.-% bezogen auf das Gewicht der jeweiligen Rezeptur.

Es wurden folgende Cremegrundlagen gemäß Tabelle 1 nach einem bekanntem Verfahren hergestellt:

| Tabelle 1: Rezeptur der Cremegrundlage | E1 | E2 | V1 | V2 |
|---|---|---|---|---|
| Hydrenol^{®} D ¹ | 12,00 | 12,00 | 12,00 | 12,00 |
| Lorol^{®} techn. ² | 2,40 | 2,40 | 2,40 | 2,40 |
| Texapon^{®} NSO F ³ | 26,50 | 26,50 | 26,50 | 26,50 |
| Stabylen^{®} 30 ⁴ | 0,20 | 0,20 | 0,20 | 0,20 |
| Cetiol^{®} OE ⁵ | 4,90 | 4,90 | 4,90 | 4,90 |
| Ammoniumsulfat | 1,00 | 1.00 | 1,00 | 1,00 |
| Turpinal^{®} SL ⁶ | 0,20 | 0.20 | 0,20 | 0,20 |
| p-Toluylendiamin H₂SO₄ | - | 1,43 | - | 1,43 |
| Resorcin | - | 0,55 | - | 0,55 |
| 2-Methylresorcin | - | 0,15 | - | 0,15 |
| 3-Amino-2-methylamino-6-methoxypyridin | - | 0,04 | - | 0,04 |
| Hydrovance^{® 7} | 3,00 | 3.00 | - | - |
| Natronwasserglas 40/42 ⁸ | 0,50 | 0,50 | 0,50 | 0,50 |
| Gluadin^{®} W40 ⁹ | 0,35 | 0,35 | 0,35 | 0,35 |
| Ammoniak (25%ige wässrige Lösung) | 7,60 | 7,60 | 7,60 | 7,60 |
| Wasser | ad 100 | ad 100 | ad 100 | ad 100 |

| | | | | |
|---|---|---|---|---|
| ¹ C₁₆-C₁₈-Fettalkohol (INCI-Bezeichnung: Cetearyl alcohol) (COGNIS) ² C₁₂-C₁₈-Fettalkohol (INCI-Bezeichnung: Coconut alcohol) (COGNIS) ³ Laurylethersulfat, Natriumsalz (ca. 27,5 % Aktivsubstanz; (INCI-Bezeichnung: Sodium Laureth Sulfate) (COGNIS) ⁴ INCI-Bezeichung: Acrylates/Vinyl Isodecanoate Crosspolymer (3V Sigma) ⁵ Dioctylether (INCI-Bezeichnung: Dicaprylether) (COGNIS) ⁶ 1-Hydroxyethan-1,1-diphosphonsäure (ca. 60% Aktivsubstanz; INCI-Bezeichnung: Etidronic Acid) (Cognis) ⁷ Hydroxyethylhamstoff (ca. 50% Aktivsubstanz; INCI-Bezeichnung: Hydroxyethyl Urea) (National Starch) ⁸ INCI-Bezeichnung: Sodium Silicate (COGNIS) ⁹ Weizenproteinhydrolysat (ca 40 % Aktivsubstanz, INCI-Bezeichung: Aqua (Water), Hydrolyzed Wheat Protein, Sodium Benzoate, Phenoxyethanol, Methytparaben, Propylparaben) (COGNIS) E1: nicht-erfindungsgemäße Cremegrundlage E2: erfindungsgemäße Cremegrundlage V1: Vergleichsgrundlage 1 V2: Vergleichsgrundlage 2 | | | | |

Die Cremegrundlage E1 eignet sich als Mittel beispielsweise an sich bereits zur Volumensteigerung keratinhaltiger Fasern. Zur Dokumentation, dass die Volumensteigerung sogar unter Einfluß von Oxidationsmitteln erzielt wird, wurden folgende oxidativ wirkende Farbveränderungsmittel hergestellt:

### 1.1 Zur Herstellung der anwendungsbereiten Bleichmittel

15 g des Bleichverstärkerpulvers gemäß Tabelle 3 wurden in 60 g der Entwickler Rezeptur gemäß Tabelle 2 gegeben und anschließend 60 g einer der Cremegrundlagen E1 oder V1 dazugegeben. Das Gemisch wurde unter Rühren bis zum Erhalt einer homogenen Anwendungsmischung vermengt.

**Tabelle 2: Entwickler-Rezeptur**

| | |
|---|---|
| Dipicolinsäure | 0.10 Gew.% |
| Na₂H₂P₂O₇ | 0.03 Gew.% |
| Turpinal^{®} SL | 1.50 Gew.% |
| Texapon^{®} N 28 ¹⁰ | 2.00 Gew.% |
| Dow Coming DB 110 ¹¹ | 0.07 Gew.% |
| Aculyn^{®} 33 ¹² | 12.00 Gew.% |
| Wasserstoffperoxid | 12.00 Gew.% |
| Ammoniak (25 %ige wäßrige Lösung) | 0.65 Gew.% |
| Wasser | ad 100 |

| | |
|---|---|
| ¹⁰ Natriumlaurylethersulfat (ca. 28% Aktivsubstanz; INCI-Bezeichnung: Sodium Laureth Sulfate) (Cognis) ¹¹ nichtionogene Siliconemulsion (ca. 10% Aktivsubstanz; INCI-Bezeichnung: Dimethicone) (Dow Coming) ¹² wässrige Acrylatdispersion (ca. 28% Aktivsubstanz; INCI-Bezeichnung: Acrylates Copolymer) (Rohm & Haas) | |

**Tabelle 3: Bleichverstärkerpulver**

| | |
|---|---|
| Aerosil^{®} 200 ¹³ | 5,0 Gew.% |
| (NH₄)₂S₂O₈ | 95,0 Gew.% |

| | |
|---|---|
| ¹³ Silikagel (pyrogene Herstellung; INCI-Bezeichung: Silica) (Degussa) | |

### 1.2 Zur Herstellung der anwendungsbereiten Färbemittel

30 g einer der Cremegrundlagen E2 oder V2 wurde unter Zugabe von 30 g Entwickler Rezeptur gemäß obiger Tabelle 2 unter Erhalt eines anwendungsbereiten oxidativen Färbemittels gemischt
Verwendung der Cremegrundlage E2 lieferte ein erfindungsgemäßes Färbemittel. Dieses wurde mit dem nicht erfindungsgemäßen Färbemittel verglichen, welches unter Verwendung der Cremegrundlage V2 erhalten wurde.

### 2.0 Volumenmessungen

2 g eines zu testenden anwendungsbereiten Mittels aus Punkt 1.1 bzw. Punkt 1.2 wurde auf eine Haarsträhne (Kerling Naturhaar, europäisch, mittelbraun und ungeschädigt; im Flottenverhältnis 4 zu 1) appliziert und nach einer Einwirkungszeit von 30 Minuten wieder abgespült. Die Haarsträhne wurde getrocknet.

Anschließend wurde das Volumen der Haarsträhne mittels Ringtest bestimmt.

Der Ringtest ergab, dass das erfindungsgemäß behandelte Haar (Mittel aus Cremgrundlage E2) ein um + 50% erhöhtes Volumen im Vergleich zum Volumen der mit dem korrespondierenden nicht erfindungsgemäßen Mittel behandelten Haarsträhne aufwies.

## Patentansprüche

1. Verfahren zur Volumensteigerung keratinhaltiger Fasern, insbesondere menschlicher Haare, in dem ein kosmetisches Mittel, enthaltend mindestens ein Harnstoffderivat gemäß Formel (I), 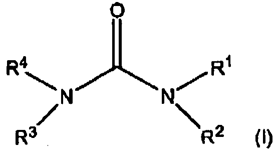 worin
die Reste R¹, R², R³ und R⁴ unabhängig voneinander für ein Wasserstoffatom, eine C₁- bis C₄-Alkylgruppe, eine C₂- bis C₅-Alkenylgruppe oder eine C₂- bis C₆-Hydroxyalkylgruppe mit mindestens einer Hydroxygruppe steht,
mit der Maßgabe, dass mindestens einer der besagten Reste eine C₂- bis C₆-Hydroxyalkylgruppe mit mindestens einer Hydroxygruppe bedeutet,
auf die keratinhaltigen Fasern aufgetragen und gegebenenfalls nach einer Einwirkungszeit wieder abgespült wird,
**dadurch gekennzeichnet,**
**daß** das kosmetische Mittel zusätzlich als farbverändernde Komponente mindestens ein Oxidationsfarbstoffvorprodukt vom Typ der Entwicklerkomponenten und gegebenenfalls zusätzlich mindestens eine Kupplerkomponente
und mindestens ein Oxidationsmittel enthält.

2. Verfahren nach Anspruch 1, **dadurch gekennzeichnet, dass** die Harnstoffderivate gemäß Formel (I) mindestens eine C₂- bis C₆-Hydroxyalkylgruppe mit mindestens einer Hydroxygruppe enthalten, welche aus mindestens einem Vertreter aus der Gruppe aus 2-Hydroxyethyl, 2-Hydroxy-2-methylprop-2-yl, 1,3-Dihydroxy-2-methylprop-2-yl, 1,3-Dihydroxyprop-2-yl, Tris(hydroxymethyl)methyl, 1,3-Dihydroxy-2-hydroxymethylprop-2-yl, 2,3-Dihydroxypropyl, 2-Hydroxypropyl, 3-Hydroxypropyl, 4-Hydroxybutyl, 1-Hydroxy-2-methylprop-2-yl, 2,3,4,5,6-Pentahydroxyhexyl und 1,3,4,5,6-Pentahydroxyhex-2-yl ausgewählt wird.

3. Verfahren nach einem der Ansprüche 1 oder 2, **dadurch gekennzeichnet, dass** gemäß Formel (I) genau einer der Reste R¹ bis R⁴ eine C₂- bis C₆-Hydroxyalkylgruppe mit mindestens einer Hydroxygruppe bedeutet

4. Verfahren nach einem der Ansprüche 1 bis 3, **dadurch gekennzeichnet, daß** als Harnstoffderivat gemäß Formel (I) mindestens eine Verbindung aus der Gruppe N-(2-Hydroxyethyl)harnstoff, N-(3-Hydroxypropyl)harnstoff, N-(2-Hydroxypropyl)harnstoff, N-(2-Hydroxy-2-methyl-prop-2-yl)harnstoff, N-(1,3-Dihydroxy-2-methyl-prop-2-yl)harnstoff und N-(1,3-Dihydroxy-2-hydroxymethyl-prop-2-yl)harnstoff verwendet wird.

5. Mittel, enthaltend in einem kosmetischen Träger eine Kombination aus mindestens einem Harnstoffderivat gemäß Formel (I) 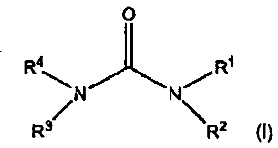 wobei die Reste R¹, R², R³ und R⁴ wie in den Ansprüchen 1 bis 4 definiert sind,
und zusätzlich als farbverändernde Komponente
mindestens ein Oxidatlonsfarbstoffvorprodukt vom Typ der Entwicklerkomponenten und gegebenenfalls zusätzlich mindestens eine Kupplerkomponente und mindestens ein Oxidationsmittel.

6. Mittel nach Anspruch 5, **dadurch gekennzeichnet, daß** es die Harnstoffderivate gemäß Formel (I) in einer Menge von 0,05 bis 5 Gew.-% bezogen auf das Gewicht des anwandungsbereiten Mittels enthält.

7. Mittel nach einem der Ansprüche 5 oder 6, **dadurch gekennzeichnet, daß** es zusätzlich mindestens ein Tensid enthält.

## Claims

1. Method for enhancing the volume of keratin-containing fibres, especially human hair, in which method a cosmetic agent, comprising at least one urea derivative according to Formula (I), 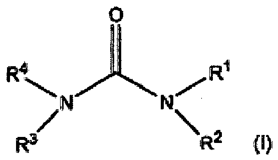 in which
the residues R¹, R2, R3 and R4 independently of each other stand for a hydrogen atom, a C1 to C4 alkyl group, a C2 to C6 alkenyl group or a C2 to C6 hydroxyalkyl group with at least one hydroxy group,
with the proviso that at least one of the stated residues means a C2 to C6 hydroxyalkyl group with at least one hydroxy group,
is applied onto the keratin-containing fibres and is optionally washed out again after a contact time,
**characterised in that**,
the cosmetic agent additionally comprises at least one oxidation dye precursor of the type of the developer components as the colour changing component and optionally additionally comprises at least one coupler component and at least one oxidising agent.

2. Method according to claim 1, **characterised in that** the urea derivatives according to Formula (I) comprise a C2 to C6 hydroxyalkyl group with at least one hydroxy group which is selected from at least one representative of the group of 2-hydroxyethyl, 2-hydroxy-2-methylprop-2-yl, 1,3-dihydroxy-2-methylprop-2-yl, 1,3-dihydroxyprop-2-yl, tris(hydroxymethyl)methyl, 1,3-dihydroxy-2-hydroxymethylprop-2-yl, 2,3-dihydroxypropyl, 2-hydroxypropyl, 3-hydroxypropyl, 4-hydroxybutyl, 1-hydroxy-2-methylprop-2-yl, 2,3,4,5,6-pentahydroxyhexyl and 1,3,4,5,6-pentahydroxyhex-2-yl.

3. Method according to one of claims 1 or 2, **characterised in that** according to Formula (I) exactly one of the R1 to R4 groups means a C2 to C6 hydroxyalkyl group with at least one hydroxy group.

4. Method according to one of claims 1 to 3, **characterised in that** at least one compound from the group N-(2-hydroxyethyl)urea, N-(3-hydroxypropyl)urea, N-(2-hydroxypropyl)urea, N-(2-hydroxy-2-methyl-prop-2-yl)urea, N-(1,3-dihydroxy-2-methyl-prop-2-yl)urea and N-(1,3-dihydroxy-2-hydroxymethyl-prop-2-yl)urea is used as the urea derivative according to Formula (I).

5. Agent, comprising in a cosmetic carrier a combination of at least one urea derivative according to Formula (I), 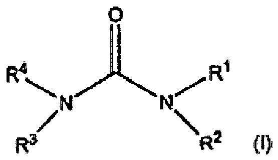 wherein the groups R1, R2, R3 and R4 are defined as in the claims 1 to 4,
and additionally as the colour changing component
at least one oxidation dye precursor of the type of the developer components and in addition optionally at least one coupler component and at least one oxidising agent.

6. Agent according to claim 5, **characterised in that** it comprises the urea derivative according to Formula (I) in an amount of 0.05 to 5 wt %, based on the weight of the ready for use agent.

7. Agent according to one of claims 5 or 6, **characterised in that** it additionally comprises at least one surfactant.

## Revendications

1. Procédé pour augmenter le volume de fibres kératiniques, en particulier de cheveux humains, dans lequel on applique sur les fibres kératiniques, un agent cosmétique comprenant au moins un dérivé d'urée répondant à la formule (I) 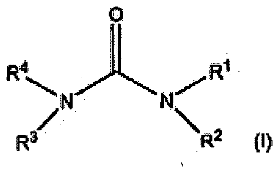 dans laquelle
les résidus R¹, R², R³ et R⁴ représentent, indépendamment l'un de l'autre, un atome d'hydrogène, un groupe alkyle en C₁-C₄, un groupe alcényle en C₂-C₆ ou un groupe hydroxyalkyle en C₂-C₆ comprenant au moins un groupe hydroxyle ;
sous réserve qu'au moins un desdits résidus représente un groupe hydroxyalkyle en C₂-C₆ comprenant au moins un groupe hydroxyle, et, de manière facultative après un temps de réaction, on l'en élimine par rinçage ;
**caractérisé en ce que** l'agent cosmétique comprend en outre, à titre de composant modifiant la couleur, au moins un précurseur de colorant d'oxydation du type des composants développeurs et, de manière facultative, en outre au moins un composant coupleur, et au moins un agent d'oxydation.

2. Procédé selon la revendication 1, **caractérisé en ce que** les dérivés d'urée répondant à la formule (I) comprennent au moins un groupe hydroxyalkyle en C₂-C₆ comprenant au moins un groupe hydroxyle, qui est choisi parmi au moins un représentant du groupe constitué par un groupe 2-hydroxyéthyle, un groupe 2-hydroxy-2-méthylprop-2-yle, un groupe 1,3-dihydroxy-2-méthylprop-2-yle, un groupe 1,3-dihydroxyprop-2-yle, un groupe tri(hydroxyméthyl)méthyle, un groupe 1,3-dihydroxy-2-hydroxyméthylprop-2-yle, un groupe 2,3-dihydroxypropyle, un groupe 2-hydroxypropyle, un groupe 3-hydroxypropyle, un groupe 4-hydroxybutyle, un groupe 1-hydroxy-2-méthylprop-2-yle, un groupe 2,3,4,5,6-pentahydroxyhexyle et un groupe 1,3,4,5,6-pentahydroxyhex-2-yle.

3. Procédé selon l'une quelconque des revendications 1 ou 2, **caractérisé en ce que**, conformément à la formule (I), précisément un des résidus R¹, R², R³ et R⁴ représente un groupe hydroxyalkyle en C₂-C₆ comprenant au moins un groupe hydroxyle.

4. Procédé selon l'une quelconque des revendications 1 à 3, **caractérisé en ce qu'**on utilise, à titre de dérivé d'urée répondant à la formule (I), au moins un composé choisi parmi le groupe comprenant la N-(2-hydroxyéthyl)urée, la N-(3-hydroxypropyl)urée, la N-(2-hydroxy-propyl)urée, la N-(2-hydroxy-2-méthyl-prop-2-yl)urée, la N-(1,3-dihydroxy-2-méthyl-prop-2-yl)urée et la N-(1,3-dihydroxy-2-hydroxy-méthyl-prop-2-yl)urée.

5. Agent comprenant, dans un support cosmétique, une combinaison d'au
moins un dérivé d'urée répondant à la formule (I) 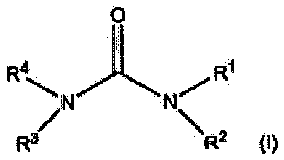 dans laquelle
les résidus R¹, R², R³ et R⁴ sont tels que définis aux revendications 1 à 4, et en outre, à titre de composant modifiant la couleur,
d'au moins un précurseur de colorant d'oxydation du type des composants développeurs et de manière facultative en outre d'au moins un composant coupleur, et d'au moins un agent d'oxydation.

6. Agent selon la revendication 5, **caractérisé en ce qu'**il comprend les dérivés d'urée répondant à la formule (I) en une quantité de 0,05 à 5 % en poids, rapportés au poids de l'agent prêt à l'emploi.

7. Agent selon l'une quelconque des revendications 5 ou 6, **caractérisé en ce qu'**il comprend en outre au moins un agent tensioactif.
